(19) Europäisches Patentamt

European Patent Office

Office européen des brevets

(11) **EP 1 440 975 A1**

(12) **EUROPEAN PATENT APPLICATION**
published in accordance with Art. 158(3) EPC

(43) Date of publication:
28.07.2004 Bulletin 2004/31

(51) Int Cl.7: **C07H 15/04**

(21) Application number: 02777867.9

(22) Date of filing: 17.10.2002

(86) International application number:
**PCT/JP2002/010797**

(87) International publication number:
**WO 2003/037907 (08.05.2003 Gazette 2003/19)**

(84) Designated Contracting States:
**AT BE BG CH CY CZ DE DK EE ES FI FR GB GR IE IT LI LU MC NL PT SE SK TR**

(30) Priority: 31.10.2001 JP 2001335496

(71) Applicant: **KABUSHIKI KAISHA UENO SEIYAKU OYO KENKYUSHO**
**Osaka-shi, Osaka 541-8543 (JP)**

(72) Inventors:
• **UENO, Ryuzo**
**Nishinomiya-shi, Hyogo 662-0038 (JP)**
• **TABATA, Akihiko**
**Kawanishi-shi, Hyogo 666-0122 (JP)**
• **HONDA, Junya,**
**502, Nishinomiyanajio-Windyhills**
**Nishinomiya-shi, Hyogo 669-1133 (JP)**
• **FURUKAWA, Yojiro**
**Itami-shi, Hyogo 664-0851 (JP)**
• **KURIYAMA, Yoshiaki**
**Kawanishi-shi, Hyogo 666-0026 (JP)**

(74) Representative: **Cresswell, Thomas Anthony et al**
**J.A. KEMP & CO.**
**14 South Square**
**Gray's Inn**
**London WC1R 5JJ (GB)**

(54) **MALTITOL CRYSTAL CONTAINING CRYSTAL OF SACCHARIDE EXCEPT MALTITOL AND METHOD FOR PRODUCTION THEREOF**

(57)    Maltitol crystals containing sugar crystals other than maltitol which have excellent solubility, almost no hygroscopicity, do not become bulky when they are stored or transported because they have a large bulk density, a small difference between packed bulk density and loose bulk density thereof and excellent flowability.

The maltitol crystals containing sugar crystals other than maltitol have a loose bulk density of particles having a particle size of 20 to 50 mesh after grinding and classification of more than 0.750 g/cc and 0.850 g/cc or less.

FIG. 2

**Description**

Detailed Description of the Invention

Field of the Invention

The present invention relates to maltitol crystals containing sugar crystals other than maltitol.

Description of the Prior Art

Since maltitol is hardly digested and absorbed in a digestive organ and rarely fermented by oral bacteria, it is used in low-calorie foods, diet foods, little cariogenic foods and sweetening agents for diabetics and the like. However, as a maltitol dried product is remarkably hygroscopic and deliquescent and hardly powdered, it is very difficult to handle.
To solve these problems, there have been proposed many technologies for crystallizing or powdering maltitol. The technologies include, for example, a crystalline mixture solid containing maltitol disclosed by Japanese Patent No. 3166102.
This patent discloses a maltitol having an apparent specific gravity of 0.72 and an oil absorptivity of 7.5 % when it has a particle size of about 20 mesh and having an apparent specific gravity of 0.61 and an oil absorptivity of 16.5 % when it has a particle size of about 50 mesh as well as its production process using an extruder having a plurality of continuous slender cooling and kneading zones and a seed crystal.
Conventional crystalline mixture solid containing maltitol have problems such as high hygroscopicity because sugar components other than maltitol such as sorbitol contained in a maltitol aqueous solution as a raw material are not crystallized. To reduce the hygroscopicity, the purity of the maltitol aqueous solution as the raw material must be increased and steps for separation by chromatography, crystallization and the like are required for this purpose, thereby making it impossible to avoid a reduction in production efficiency.
Although the crystalline mixture solid containing maltitol disclosed by the above patent has a relatively large apparent specific gravity, it is unsatisfactory and needs a large space or a transporting tool when it is stored or transported.

Summary of the Invention

It is an object of the present invention to provide maltitol crystals containing sugar crystals other than maltitol, which have solved the physical problems and production problems of conventional crystalline mixture solid containing maltitol, and a production process therefor.
It is another object of the present invention to provide maltitol crystals containing sugar crystals other than maltitol, which have excellent solubility and almost no hygroscopicity, and a production process therefor.
It is still another object of the present invention to provide maltitol crystals containing sugar crystals other than maltitol, which do not become bulky when they are stored or transported because they have a large bulk density, and a production process therefor.
It is a further object of the present invention to provide maltitol crystals containing sugar crystals other than maltitol, which have a small difference between packed bulk density and loose bulk density thereof and high flowability and a production process therefor.
It is a still further object of the present invention to provide a maltitol crystal mixture which has high flowability and whose caking during storage is markedly suppressed.
Other objects and advantages of the present invention will become apparent from the following description.

Means for solving the Problems

According to the present invention, the above objects and advantages of the present invention are attained by maltitol crystals containing sugar crystals other than maltitol, which have a loose bulk density of particles having a particle size of 20 to 50 mesh after grinding and classification of more than 0.750 g/cc and 0. 850 g/cc or less.
The present invention will be described in detail hereinunder.
The maltitol crystals containing sugar crystals other than maltitol of the present invention have a loose bulk density of particles having a particle size of 20 to 50 mesh after grinding and classification of preferably more than 0.760 g/cc and 0.840 g/cc or less, more preferably more than 0.770 g/cc and 0.830 g/cc or less. This loose bulk density is a value measured with the PT-N powder tester (of Hosokawa Micron Co., Ltd.).
Out of the above maltitol crystals containing sugar crystals other than maltitol, what contain 0.5 to 20 wt% of sorbitol are preferred and what contain 2 to 15 wt% of sorbitol are more preferred. Further, the sorbitol is preferably sorbitol crystals.

The maltitol crystals containing sugar crystals other than maltitol of the present invention have a compressibility of particles having a particle size of 20 to 50 mesh after grinding and classification of preferably 0 to 7 %, more preferably 0 to 4 % because they have excellent flowability.

When the above maltitol crystals containing sugar crystals other than maltitol are observed through a scanning electron microscope at a magnification of X1,000, needle-like crystals can be confirmed.

Since sugar components other than maltitol such as sorbitol are crystallized in the maltitol crystals containing sugar crystals other than maltitol of the present invention, the hygroscopicity of the maltitol crystals is much lower than that of conventional crystalline mixture solid containing maltitol.

The oil absorptivity of the above maltitol crystals containing sugar crystals other than maltitol is more than 0.1 wt% and less than 7.0 wt%, preferably more than 0.5 wt% and less than 6.5 wt% when they are ground and classified to achieve a particle size of 20 to 50 mesh. This oil absorptivity is a value calculated from the weight (A) of a sample containing the residual oil based on the following expression. This sample is obtained by mixing 15 g of a sample prepared by grinding and classification to achieve a particle size of 20 to 50 mesh with a suitable amount of castor oil, leaving the resulting mixture at room temperature for 5 minutes, and centrifuging a centrifugal tube having a 80-mesh net stretched thereon by a centrifugal machine (1,300 G, 10 minutes) to remove an oil fraction which was not retained in the sample.

After the above sample and castor oil are mixed together in the 80-mesh net shaped like a bag, the resulting mixture may be placed in the tube having a grating therein.

$$\text{Oil absorptivity (wt\%)} = (A - 15)/15 \times 100$$

A preferred example of the process for producing the maltitol crystals containing sugar crystals other than maltitol of the present invention is shown below. The maltitol crystals containing sugar crystals other than maltitol of the present invention are not limited by the production process.

Preferably, the raw material for producing the maltitol crystals containing sugar crystals other than maltitol of the present invention has a maltitol content (based on the solid content) of 70 to 95 wt%, a sorbitol content (based on the solid content) of 2.5 to 15 wt% and a maltotriitol and higher polymerized sugar alcohol total content (based on the solid content) of 2.5 to 15 wt% . More preferably, it has a maltitol content (based on the solid content) of 80 to 93 wt%, a sorbitol content (based on the solid content) of 3.5 to 10 wt% and a maltotriitol and higher polymerized sugar alcohol total content (based on the solid content) of 3.5 to 10 wt%.

The maltitol aqueous solution may have any water content if sorbitol is in a supersaturated state. Therefore, the maltitol aqueous solution may be concentrated until sorbitol becomes supersaturated. The concentration method is not limited and a general concentration method such as vacuum concentration or thin film concentration may be employed.

In the production of the maltitol crystals containing sugar crystals other than maltitol of the present invention, a device and machine of any type, for example, an open or closed type, or a batch or continuous type may be used if it can knead and cool the raw material at the same time. A continuous kneader or extruder which can extrude the raw material from an outlet port continuously after kneading and cooling is preferably used. Examples of the kneader include an extruder, continuous kneader, mixtron and kneedex, Out of these, an extruder is preferred. Examples of the extruder include the KRC kneader (of Kurimoto, Ltd.), double-screw extruder for foods (of Nippon Steel Co., Ltd.) and double-screw cooking extruder (of W & P AG of Germany).

The temperature when the raw material is supplied into the kneader is preferably about 100 to 135°C. The temperature of a cooling portion for forming a plastic mass may be adjusted to a temperature at which generated crystallization heat can be removed, preferably 90° C or less, more preferably 50°C or less.

The feed rate of the raw material, which differs according to the type and capacity of the kneader in use, is preferably about 2 to 30 kg/hr when the KRC kneader (2S) of Kurimoto, Ltd. is used.

The obtained crystals can be made powdery by grinding or granular by granulation. Methods for grinding and granulation are not particularly limited and a commonly used grinder and granulator may be used. If necessary, the obtained powder or granule may be dried by a commonly used drying method or sifted. When it is to be dried, the drying method is selected from pneumatic coveying drying, fluidized bed drying, vacuum drying and tray drying all of which are generally used.

When the magma is to be extruded from a continuous type kneader, the shape of the magma may be arbitrary, for example, noodle-like, ribbon-like, rod-like or plate-like shape. In consideration of the subsequent steps such as cooling and grinding, it is preferably extruded in a noodle-like or ribbon-like shape. A punching plate mounted to the outlet port preferably has an opening diameter of about 2 to 5 mm and an opening ratio of about 10 to 40 %.

The cooling method is not particularly limited. For example, the magma extruded from the kneader may be directly exposed to cold air, left at room temperature or cooled to room temperature with cold air on a metal net belt. However, when the magma is quickly cooled to such an extent that its surface temperature drop speed is faster than 10°C/min, needle-like crystals hardly grow disadvantageously.

When the maltitol crystals containing sugar crystals other than maltitol of the present invention satisfy the above conditions, high-quality powdery or granular maltitol crystals containing sugar crystals other than maltitol, which do not need a drying step, are easy to handle and soluble, and have almost no hygroscopicity can be obtained at a low cost in a short period of time.

Further, a maltitol crystal mixture is obtained by mixing cane sugar with the maltitol crystals containing sugar crystals other than maltitol of the present invention. The cane sugar is not particularly limited and may be granulated sugar, refined white soft sugar, brown soft sugar or the like. Out of these, granulated sugar is preferred from the viewpoints of the flowability and caking properties of the mixture. The mixing ratio of the cane sugar can be suitably adjusted according to purpose and not limited. However, when the mixing ratio of the maltitol crystals containing sugar crystals other than maltitol to cane sugar is 5:95 to 50:50, preferably 10:90 to 30:70, more preferably 15:85 to 20:80, the taste of the mixture are well balanced and it is easy to use the mixture.

The maltitol crystals containing sugar crystals other than maltitol of the present invention will be described in more detail with reference to Examples hereinafter.

In Examples, "%" means "wt%" unless otherwise stated.

Examples

Example 1

A maltitol aqueous solution (containing maltitol = 90 %, sorbitol = 5 %, maltotriitol and higher polymerized sugar alcohol = 5 %, water content = 1.5 %, solids content = 98.5 %, temperature = 128°C) was continuously supplied into a continuous kneader having slender kneading and cooling zones (S-2 KRC kneader of Kurimoto, Ltd. , 60 rpm, jacket temperature = 70° C) at a rate of 5 kg/h, kneaded while air was blown into the aqueous solution to disperse air bubbles into the aqueous solution and then kept kneaded and cooled. A noodle-like solid was discharged from a punching plate at the outlet. This solid (surface temperature = about 70° C) was cooled with 15° C cold air for 10 minutes to reduce its surface temperature to about 30°C and ground to obtain high-quality maltitol crystals containing sugar crystals other than maltitol.

After the obtained powders were further ground and classified to achieve a particle size of 20 to 50 mesh, the bulk density (loose bulk density and packed bulk density), compressibility, oil absorptivity and sorbitol crystal content of the obtained powders were measured. The results are shown in Table 1.

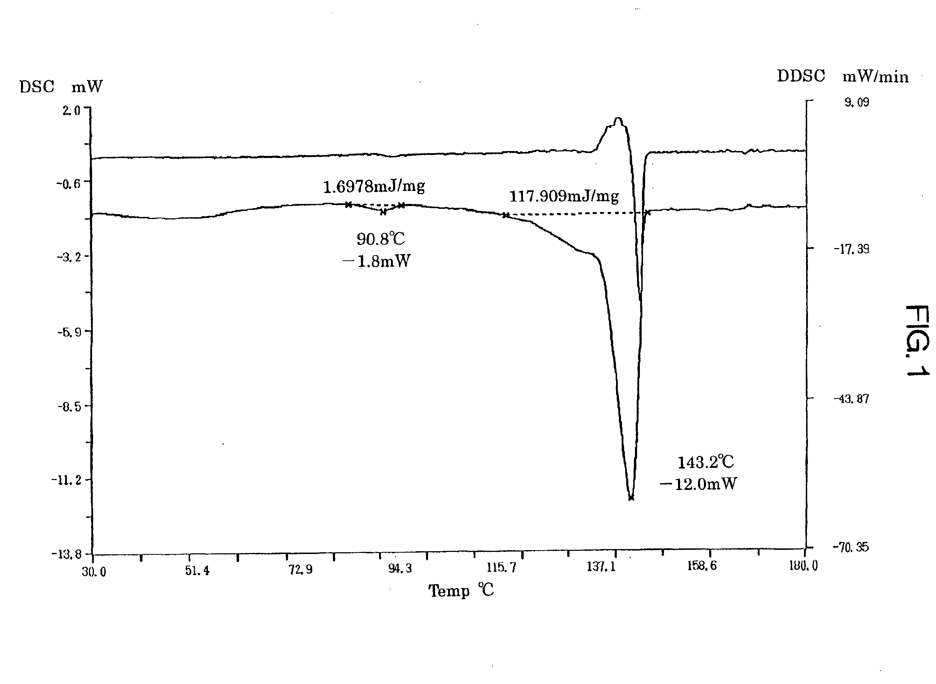

The DSC chart of the obtained powders is shown in Fig. 1.

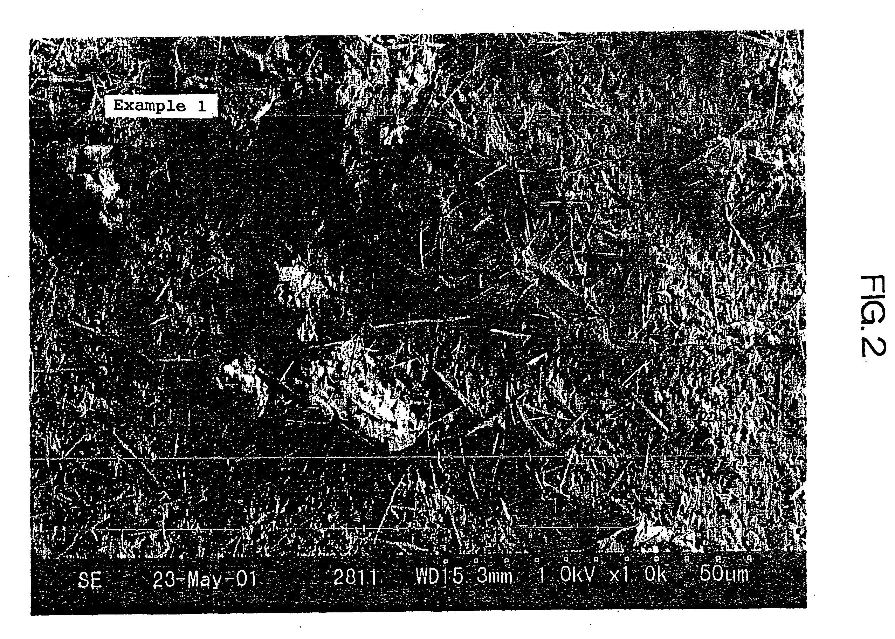

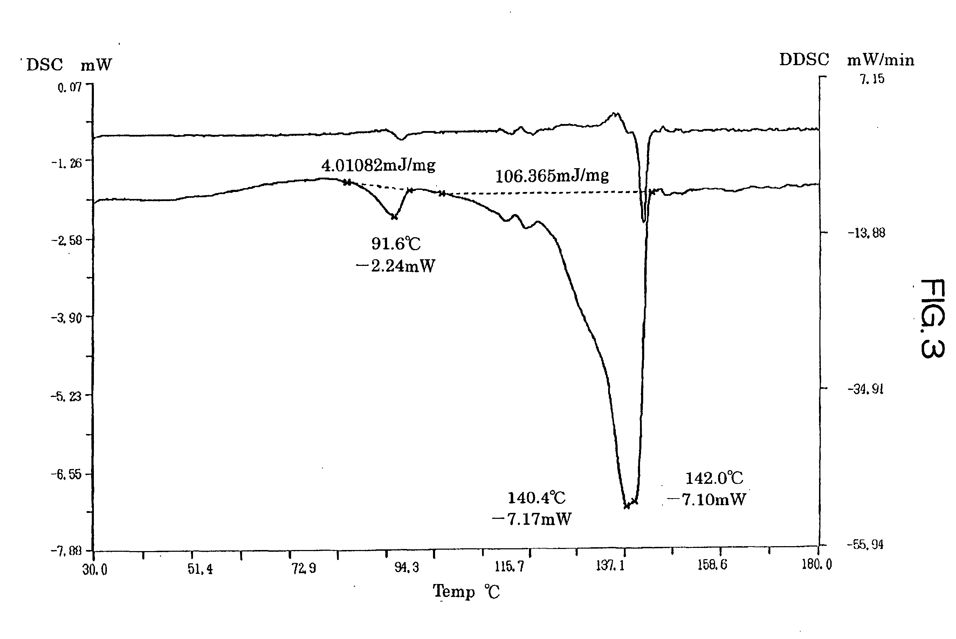

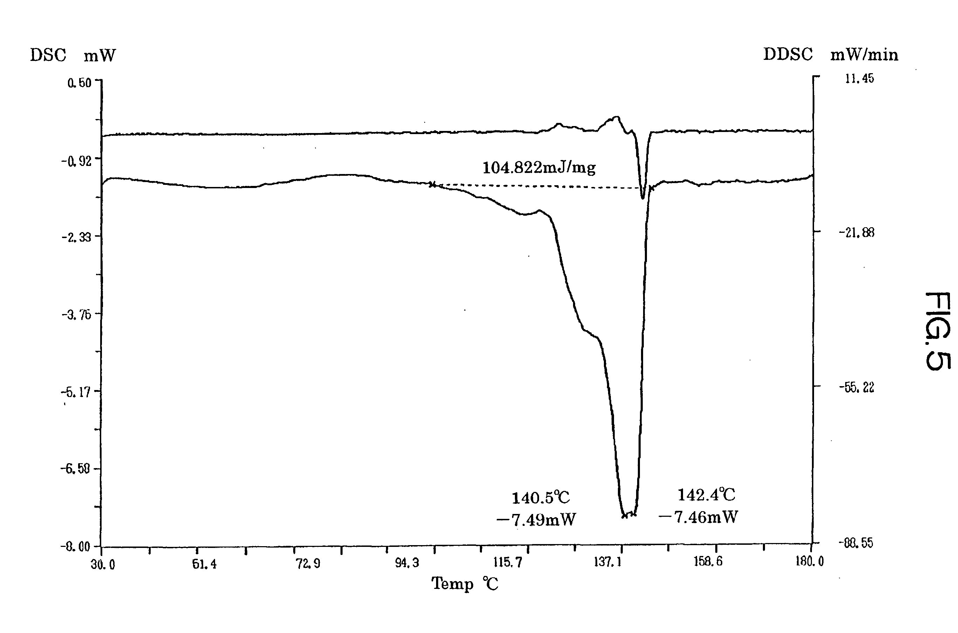

The DSC measurement conditions are given below (the same shall be applied to Fig. 3 and Fig. 5).

Sample (obtained powders): 10 mg

Reference: A1 open pan 10 mg

| Temperature program: | | | | |
|---|---|---|---|---|
| | ° C | ° C/ min | min | sec |
| 1 | 25-30 | 10 | 0.1 | 0.5 |
| 2 | 30-200 | 4 | 0 | 0.5 |

Further, when the obtained powders were observed through an electron microscope, needle-like crystals could be confirmed. The results are shown in Fig. 2.

Example 2

Production was carried out under the same conditions as in Example 1 until a noodle-like solid was discharged and the discharged solid was cooled with 20°C cold air for 20 minutes to reduce its surface temperature to about 30° C and ground to obtain high-quality maltitol crystals containing sugar crystals other than maltitol.

After the obtained powders were further ground and classified to achieve a particle size of 20 to 50 mesh, the bulk density (loose bulk density, packed bulk density), oil absorptivity and sorbitol crystal content of the obtained powders were measured. The results are shown in Table 1. The DSC chart of the obtained powders is shown in Fig. 3.

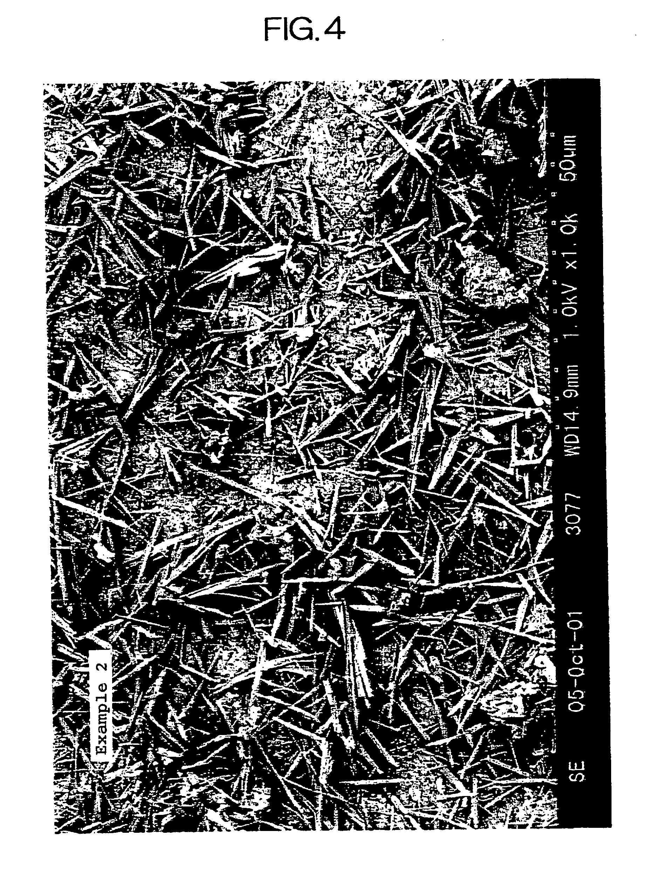

Further, when the obtained powders were observed through an electron microscope, needle-like crystals could be confirmed. The results are shown in Fig. 4.

Comparative Example 1

The production was carried out under the same conditions as in Example 1 until a noodle-like solid was discharged from the punching plate at the outlet, and the discharged solid was cooled with 5° C cold air for 2 minutes to reduce its surface temperature to about 30°C and ground to obtain crystalline mixture solid containing maltitol.

After the obtained powders were further ground and classified to achieve a particle size of 20 to 50 mesh, the bulk density (loose bulk density, packed bulk density), oil absorptivity and sorbitol crystal content of the powders were measured. The results are shown in Table 1. The DSC chart of the obtained powders is shown in Fig. 5.

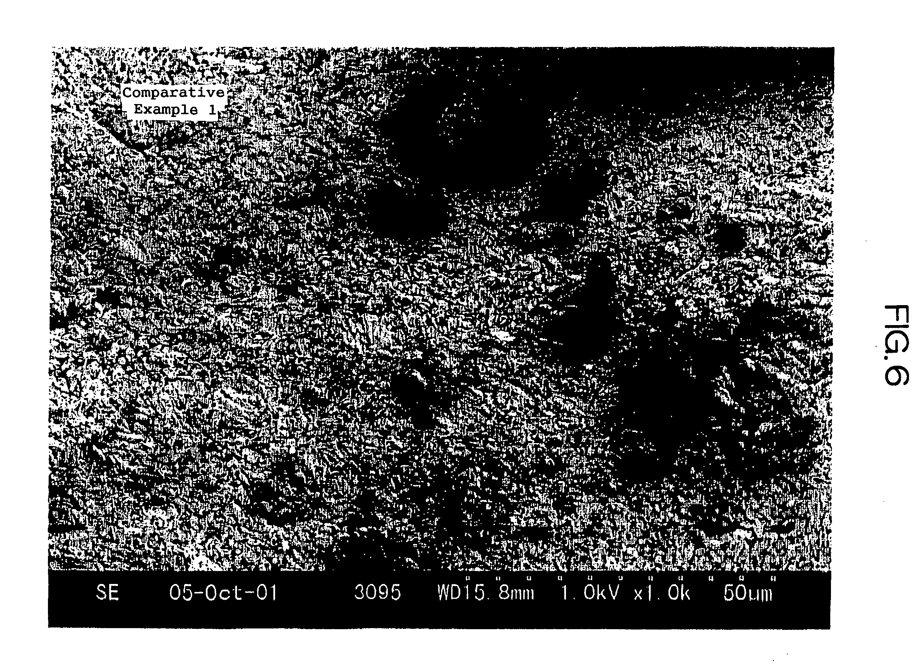

Further, when the obtained powders were observed through an electron microscope, a needle-like crystal could not be confirmed. The results are shown in Fig. 6.

## Table 1

|  | Ex.1 (20 to 50 mesh) | Ex.2 (20 to 50 mesh) | C.Ex.1 (20 to 50 mesh) |
|---|---|---|---|
| Loose bulk density (g/cc) | 0.784 | 0.792 | 0.711 |
| Packed bulk density (g/cc) | 0.793 | 0.807 | 0.718 |
| Compressibility (%) | 1.1 | 1.9 | 1.0 |
| Oil absorptivity (wt%) | 5.8 | 5.7 | 5.5 |
| Sorbitol crystal content (wt%) | 0.9 | 2.1 | 0.0 |

Ex.: Example      C.Ex.: Comparative Example

The measurements of the following physical property values and the observation through an electron microscope of Examples and Comparative Examples were carried out as follows.

loose bulk density (g/cc)

This was measured with the PT-N powder tester (of Hosokawa Micron Co., Ltd.).

packed bulk density (g/cc)

This was measured by setting the number of tapping times to 180 and using the PT-N powder tester (of Hosokawa Micron Co., Ltd.).

compressibility (%)

This was calculated from the following expression.

$$\text{Compressibility (\%)} = (\text{packed bulk density} - \text{loose bulk density})/(\text{packed bulk density}) \times 100$$

oil absorptivity (wt%)

15 g of a sample obtained by grinding and classification to achieve a particle size of 20 to 50 mesh and a suitable amount of castor oil were mixed together and left at room temperature for 5 minutes, an oil fraction which was not retained in the sample was removed by a centrifugal machine (1300 G, 10 minutes) having a 80-mesh net stretched, and the weight (A) of the sample containing the residual oil was measured. The oil absorptivity was calculated from this value based on the following equation.

$$\text{oil absorptivity (wt\%)} = (A - 15)/15 \times 100$$

sorbitol crystal content (wt%)

Each sample was dried at normal temperature under vacuum for 1 hour and placed in a closed sample container (made from Al and having a capacity of 15 μl) to measure the enthalpy (ΔH) of a peak which appeared at around 90° C under conditions such as a temperature range of 30 to 200°C and a temperature elevation rate of 4° C/min with a differential scanning thermometer (DSC6200 of Seiko Instruments Co., Ltd. ). The sorbitol crystal content was calculated from the obtained chart based on the following expression.

$$\text{sorbitol crystal content (wt\%)} = (\Delta H \text{ of a peak at around}$$

$$90°C)/(\Delta H \text{ of pure sorbitol crystal}) \times 100$$

* ΔH of pure sorbitol crystal = 200 (J/g)

observation through electron microscope

Pt-Pb was deposited on the sample for 60 seconds and the obtained sample was observed through a scanning electron microscope (S-4300 of Hitachi, Ltd.) at a voltage of 1 kV and a magnification of X1,000.

Effect of the Invention

As described above, according to the present invention, there are provided maltitol crystals containing sugar crystals other than maltitol, which have excellent solubility, almost no hygroscopicity, do not become bulky when they are stored and transported because they have a large bulk density, a small difference between packed bulk density and loose bulk density thereof and excellent flowability.

Brief Description of the Drawings

Fig. 1 is a DSC chart of maltitol crystal powders containing sugar crystals other than maltitol obtained in Example 1;
Fig. 2 is a scanning electron microphotograph of maltitol crystal powders containing sugar crystals other than maltitol obtained in Example 1;
Fig. 3 is a DSC chart of maltitol crystal powders containing sugar crystals other than maltitol obtained in Example 2;
Fig. 4 is a scanning electron microphotograph of maltitol crystal powders containing sugar crystals other than maltitol obtained in Example 3;
Fig. 5 is a DSC chart of crystalline mixture solid containing maltitol powders obtained in Comparative Example 1; and
Fig. 6 is a scanning electron microphotograph of crystalline mixture solid containing maltitol powders obtained in Comparative Example 1.

**Claims**

1.    Maltitol crystals containing sugar crystals other than maltitol, which have a loose bulk density of particles having a particle size of 20 to 50 mesh after grinding and classification of more than 0.750 g/cc and 0.850 g/cc or less.

2.    The maltitol crystals containing sugar crystals other than maltitol according to claim 1, which have a loose bulk density of particles having a particle size of 20 to 50 mesh after grinding and classification of more than 0.760 g/cc and 0.840 g/cc or less.

3.    The maltitol crystals containing sugar crystals other than maltitol according to claim 1, which have a loose bulk density of particles having a particle size of 20 to 50 mesh after grinding and classification of more than 0.770 g/cc and 0.830 g/cc or less.

4.    The maltitol crystals containing sugar crystals other than maltitol according to any one of claims 1 to 3, which contain 0.5 to 20 wt% of sorbitol.

5.    The maltitol crystals containing sugar crystals other than maltitol according to claim 4, wherein the sorbitol is sorbitol crystals.

**6.** The maltitol crystals containing sugar crystals other than maltitol according to any one of claims 1 to 5, which contain needle-like crystals observed through a scanning electron microscope at a magnification of X1,000.

**7.** The maltitol crystals containing sugar crystals other than maltitol according to any one of claims 1 to 6, which have an oil absorptivity of particles having a particle size of 20 to 50 mesh after grinding and classification of more than 0.1 wt% and less than 7.0 wt%.

**8.** The maltitol crystals containing sugar crystals other than maltitol according to claim 7, which have an oil absorptivity of particles having a particle size of 20 to 50 mesh after grinding and classification of more than 0.5 wt% and less than 6.5 wt%.

**9.** The maltitol crystals containing sugar crystals other than maltitol according to any one of claims 1 to 8, which have a compressibility of particles having a particle size of 20 to 50 mesh after grinding and classification of 0 to 7 %.

**10.** A process for producing the maltitol crystals containing sugar crystals other than maltitol according to any one of claims 1 to 9, comprising the steps of:

supplying a maltitol aqueous solution whose water content has been adjusted so that sorbitol becomes supersaturated into a kneader and kneading it to produce a plastic mass; and
cooling the plastic mass after kneading in such a manner that its surface temperature drop speed does not exceed 10° C/min.

**11.** A maltitol crystal mixture obtained by mixing together the maltitol crystals containing sugar crystals other than maltitol of claim 5 and cane sugar.

**12.** The maltitol crystal mixture of claim 11, wherein the cane sugar is granulated sugar.

**13.** The maltitol crystal mixture of claim 11 or 12, wherein the mixing ratio of the maltitol crystals containing sugar crystals other than maltitol to cane sugar is 5:95 to 50:50.

# FIG. 1

FIG. 2

FIG. 3

EP 1 440 975 A1

# FIG.4

# FIG.5

# FIG.6

## INTERNATIONAL SEARCH REPORT

| | International application No. |
|---|---|
| | PCT/JP02/10797 |

A. CLASSIFICATION OF SUBJECT MATTER
    Int.Cl⁷  C07H15/04

According to International Patent Classification (IPC) or to both national classification and IPC

B. FIELDS SEARCHED

Minimum documentation searched (classification system followed by classification symbols)
    Int.Cl⁷  C07H15/04, C13K13/00

Documentation searched other than minimum documentation to the extent that such documents are included in the fields searched

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X | EP 491953 A1   (Towa Chemical Industry Co., Ltd.), 01 July, 1992 (01.07.92), Full text<br>& JP 3166102 B2          & WO 92/00309 A1<br>& AU 7891991 A          & FI 920807 A<br>& CA 2065301 A          & US 5354856 A<br>& US 5583215 A          & DE 69125806 A1 | 1-13 |
| A | EP 741140 A1   (Towa Chemical Industry Co., Ltd.), 06 November, 1996 (06.11.96),<br>& JP 9-19300 A          & AU 5192696 A<br>& DE 69617088 A1          & CN 1148046 A<br>& US 5873943 A | 1-13 |

☒ Further documents are listed in the continuation of Box C.      ☐ See patent family annex.

| | |
|---|---|
| * Special categories of cited documents:<br>"A"  document defining the general state of the art which is not considered to be of particular relevance<br>"E"  earlier document but published on or after the international filing date<br>"L"  document which may throw doubts on priority claim(s) or which is cited to establish the publication date of another citation or other special reason (as specified)<br>"O"  document referring to an oral disclosure, use, exhibition or other means<br>"P"  document published prior to the international filing date but later than the priority date claimed | "T"  later document published after the international filing date or priority date and not in conflict with the application but cited to understand the principle or theory underlying the invention<br>"X"  document of particular relevance; the claimed invention cannot be considered novel or cannot be considered to involve an inventive step when the document is taken alone<br>"Y"  document of particular relevance; the claimed invention cannot be considered to involve an inventive step when the document is combined with one or more other such documents, such combination being obvious to a person skilled in the art<br>"&"  document member of the same patent family |
| Date of the actual completion of the international search<br>    08 January, 2003 (08.01.03) | Date of mailing of the international search report<br>    21 January, 2003 (21.01.03) |
| Name and mailing address of the ISA/<br>    Japanese Patent Office | Authorized officer |
| Facsimile No. | Telephone No. |

Form PCT/ISA/210 (second sheet) (July 1998)

**INTERNATIONAL SEARCH REPORT**

| International application No. |
| --- |
| PCT/JP02/10797 |

C (Continuation). DOCUMENTS CONSIDERED TO BE RELEVANT

| Category* | Citation of document, with indication, where appropriate, of the relevant passages | Relevant to claim No. |
| --- | --- | --- |
| A | EP 816373 A1 (Towa Chemical Industry Co., Ltd.), 07 January, 1998 (07.01.98), & JP 10-17589 A  & AU 2834997 A & DE 69708395 A1  & US 5932015 A | 1-13 |
| A | GB 2097004 A (Kabushiki Kaisha Hayashibara Seibutsu Kagaku Kenkyujo), 27 October, 1982 (27.10.82), & JP 63-2439 B2  & AU 7757281 A & BE 891442 A  & SE 8106884 A & FI 813788 A  & FR 2499576 A1 & DE 3146085 A1  & US 4408041 A & CA 1186304 A  & US 4717765 A & US 4725387 A  & US 4789559 A & US 4917916 A | 1-13 |
| A | GB 1287509 A (Towa Kasei Kogyo Co., Ltd.), 31 August, 1972 (31.08.72), & JP 52-20444 B1  & BE 759609 A & DE 2059246 A1  & FR 2072535 A1 & CH 520638 A | 1-13 |

Form PCT/ISA/210 (continuation of second sheet) (July 1998)